(19) Europäisches Patentamt

European Patent Office

Office européen des brevets

(11) **EP 4 455 810 A1**

(12) **EUROPEAN PATENT APPLICATION**
published in accordance with Art. 153(4) EPC

(43) Date of publication:
**30.10.2024 Bulletin 2024/44**

(21) Application number: 22910255.3

(22) Date of filing: **17.01.2022**

(51) International Patent Classification (IPC):
*G05B 19/406* (2006.01)    *G05B 19/414* (2006.01)
*G05B 15/02* (2006.01)    *H04L 12/28* (2006.01)

(52) Cooperative Patent Classification (CPC):
**F24F 8/00; F24F 11/00; G01N 3/00; G01N 15/02;**
**G01N 15/06; G05B 15/02; G05B 19/406;**
**G05B 19/414; H04L 12/28; Y02B 30/70**

(86) International application number:
**PCT/ES2022/070013**

(87) International publication number:
**WO 2023/118621 (29.06.2023 Gazette 2023/26)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **20.12.2021 ES 202132498 U**

(71) Applicant: **Airquality Safe, S.L.**
**29590 Malaga (ES)**

(72) Inventor: **LOPEZ HIDALGO, Jose Luis**
**29590 MALAGA (ES)**

(74) Representative: **González López-Menchero,**
**Álvaro Luis**
**Protectia Patentes y Marcas, S.L.**
**C/ Almagro, 3 2o izda.**
**28010 Madrid (ES)**

(54) **AIR QUALITY SENSOR-ACTUATOR DEVICE**

(57) The present invention relates to an air quality sensor-actuator device that monitors various physico-chemical variables, controls up to two external devices and regulates by means of 0-10V control any machine that has this control input, communicates to the Internet via Wi-Fi with any 2.4GHz router and has RS485 serial connectivity for integration thereof into open source ModBus. The device measures different variables concerning the air by means of a series of sensor means, in addition to carrying out a series of calculations using processing means and, depending on the data obtained, send command and actuation signals on external devices, and it also has a human-machine interface. The sensor means that the device subject matter of the invention has are: a temperature sensor, a relative humidity sensor, a particulate matter sensor that measures particle sizes of 1 micron, 2.5 microns and 10 microns, another sensor for measuring volatile organic compounds (VOC) and a fifth sensor for measuring CO2.

EP 4 455 810 A1

**Description**

## OBJECT OF THE INVENTION

The subject matter of the present invention, as the title of the invention establishes, is a sensor device for various substances suspended in the surrounding air in such a way that it reports their presence, as well as their concentration and consequently activates the relevant actuators to improve the quality of said air in the event the latter degrades or worsens. The device subject matter of the invention has two man-machine interfaces, namely, one direct interface by having nominated and scaled indicators in a range of colours to inform and alert of quality levels, and another virtual interface by also being able to be informed by means of a computer application, which can be installed on Smartphone-type mobile terminals or on traditional computers. Connectivity with the exterior, apart from the power supply via USB connector or the conventional 230Vac electrical grid, consists of 2+1 wires under the Modbus protocol, digital and analogue outputs and digital inputs.

The present invention is characterised by the special technical features of the invention, as well as its joint arrangement and its functional interaction such that, on the one hand, it is possible to obtain 8 variables (5 measured and 3 calculated) of the surrounding atmosphere and, on the other hand, act on up to 3 devices capable of controlling these variables in case one or some of them exceed the established limits. These limits have been taken by national and international references and standards, such as the European Environment Agency (EEA), the German Environment Agency (UBA), the National Institute for Safety and Hygiene at Work (INSHT) and the American Society of Heating Refrigeration and Air Conditioning Engineers (ASHRAE).

Therefore, the present invention is limited within the field of measurement, regulation and control devices, and particularly among devices used in environmental air quality.

## BACKGROUND OF THE INVENTION

Electronic devices for measuring the different chemical and physical variables that determine temperature, density, volume or percentage exist well into the 20th century, starting with the physical temperature variable at the earliest and ending with the latest, already at the end of the 20th century, with more complex sensors capable of sensing particulate matter of up to 1 microgram per cubic meter or volatile organic matter.

Once sensors of this type were developed, they evolved into sensor-actuators, which actively participate in control or regulation. For example, sensing $CO_2$, in such a way that when a certain threshold is exceeded, a normally open contact of a relay is closed, intended to actuate a ventilation flap opening or an aeration motor.

As sensors have been miniaturised and diversified, more versatile devices have emerged, encouraged in turn by global concern due to pollution, poor environmental quality or pathogenic substances suspended in the air such as the SARS-COV-2 virus.

Although there are many developments in measuring devices, there are not so many meter-actuators, which tend to have few physical-chemical variables, the most numerous being $CO_2$ sensor-actuators, typically activating from a volumetric concentration threshold of parts per million (ppm), which is usually adjustable.

However, all existing means for controlling and regulating environmental air quality present aspects that can be improved, such as:

- Communication with other systems, by means of a RS485 serial port, being able to connect up to 246 devices or wireless connection without limit on the devices managed by the cloud posted on an Internet server.
- Multiple measurements of physical and chemical variables: Temperature, relative humidity, particulate matter of 1 micron, 2.5 microns and 10 microns (hereinafter PM1, PM2.5 and PM10 respectively), volatile organic compounds (hereinafter VOC) and $CO_2$. This ability to simultaneously measure 5 variables underpins the application for CLAIM 1.
- Calculation of the absolute humidity, dew point and thermal comfort variables. The measurements of the sensors allow us to know this set of variables as they are a function thereof and therefore are offered as additional information. Showing these 3 measurements calculated based on the values obtained from the sensors is the fundamental basis of the application for CLAIM 2.
- 2 analogue outputs to act on elements such as ventilation and/or air purification devices.
- 1 control output 0-10V, intended for regulating climate or ventilation machines, adjusting the flow thereof depending on the levels reached in the sensors.
- Measurement of the temperature of the room using a thermopile sensor, based on measuring the temperature by capturing the infrared spectrum seen by the device.
- 2 digital inputs, intended to receive external information. This information is configurable as a normally open or closed contact, with the aim of obtaining conditions of the work environment, such as air duct pressure switches, presence sensors, hotel room card cases, etc.

Therefore, the object of the present invention is to overcome, on the one hand, the drawbacks mentioned, and, on the other hand, to incorporate a novel device into the state of the art, for which, taking into account its benefits, a comparatively functional parallelism has not been found, developing a measurement, regulation and control device for the environmental air quality such as the one described below and is included in its essential nature in its claims.

## DESCRIPTION OF THE INVENTION

The subject matter of the present invention is an air quality sensor-actuator device that monitors various physical and chemical variables, controls up to 2 external devices and regulates by means of 0-10V control any machine that has this control input, communicates to the Internet via Wi-Fi with any 2.4GHz router and has RS485 serial connectivity for integration thereof into open source ModBus.

The air quality sensor-actuator is a device that, on the one hand, measures different variables concerning the air by means of a series of sensor means, in addition to carrying out a series of calculations using processing means and, depending on the data obtained, send command and actuation signals on external devices. It also has a human-machine interface to display the quality of the different measured parameters and connectivity with the outside through either a physical or wired connection or through a wireless connection.

The sensor means that the device subject matter of the invention has are: a temperature sensor, a relative humidity sensor, a particulate matter sensor that measures particle sizes of 1 micron, 2.5 microns and 10 microns, another sensor for measuring volatile organic compounds (VOC) and a fifth sensor for measuring CO2.

The processing means are responsible for calculating absolute humidity, dew point and thermal comfort.

In a possible embodiment, the command and actuation signals comprise two analogue outputs to act on a ventilation device and the other to act on an air purification device.

It also has a 0-10 Volt control output intended for climate or ventilation regulation, adjusting the flow rate thereof depending on the levels reached.

The human-machine interface for displaying the quality of the different measured parameters consists of a central circle illuminated with different colours depending on the quality of the parameters, a series of backlit acronyms that can be selected by means of a push button, where the backlit acronyms are AQ, PM, VOC and $CO_2$ that correspond to air quality, particle size, volatile organic compounds, and $CO_2$ level.

The sensor-actuator that is the subject matter of the invention is connected to the conventional 230Vac electrical grid, it also has an alternative 5V power supply via a micro USB type B female connector; an RS485 serial port with ModBus protocol including a parallel output with the input for possible Daisy Chain connectivity; a 2.4Ghz Wi-Fi connection integrated internally into the hardware of the device to connect to a standard 2.4GHz router; two digital outputs, the first is a normally open contact and the second is a dual contact that offers normally open and closed switching with respect to a common switching; an analogue output that offers a direct current in the range of 0 to 10Vdc; two digital inputs: D11, which when connected to ground interacts with the device to detect whether there is pressure in the ducts (through a pressure switch), in the event there is no pressure in the air ducts the device receives a closure of DI1 with GND and will report in the APP warning of this event, and D12 which when connected to ground (GND) opens the contacts DO1 and DO2, shutting down the machinery that is connected to these outputs. Its function is intended to cause an external general shut-down coming from, for example, a hotel room card holder, window opening sensors or a simple external general shut-down switch; a man-machine interface arranged on the front that consists of illumination by circular LED diffuser showing the measurement levels in 5 colours, this circle with white light would report any operating anomaly. A small star icon is used to inform that the contact DO2 has been closed and a level 3 or higher of VOC or PM has been reached, that is, the surrounding atmosphere of the device is being purified. it is to indicate or consult what is being measured. A push button to consult in turn the variable desired, shown on the front with the acronyms AQ, PM, VOC and CO2 in white. There are five levels to be shown in colours in all the variables, namely:

Level 1: blue colour considered excellent.
Level 2: green colour considered normal.
Level 3: yellow colour considered average.
Level 4: orange colour considered poor.
Level 5: red colour considered bad.

The firmware of the device offers a 5-colour indication of the levels by thresholds in the central circle, with blue being considered as excellent (level 1), green as normal (level 2), yellow as average (level 3), orange as poor (level 4) and red as bad (level 5). The $CO_2$ levels, VOC levels, and the worst of the particulate matter measurements PM1, PM2.5 or PM10 can be checked by using the push button. A fourth option of those available for display is the overall air quality level, named by the acronym AQ on the device and representing the worst of any of the previous variables.

The hardware of the device that is the subject matter of the invention integrates a VOC sensor, a $CO_2$ sensor,

a particulate matter sensor that measures sizes of 1, 2.5 and 10 microns, a thermopile temperature sensor and a relative humidity sensor as sensor elements inside the casing and integrated into the electronic unit. With these measurements, the device firmware, in addition to offering sensor measurements, calculates the dew point in degrees Celsius and the level of thermal comfort, divided into five levels. These comfort levels are based on the RH/T diagram (Relative humidity in percentage/Temperature in degrees Celsius) of the ANSI ASHRAE 55 standard (Thermal Environmental Conditions for Human Occupancy), published in its first edition in 1966 and constantly updated. This ANSI standard establishes ranges of indoor environmental conditions to achieve acceptable thermal comfort for building occupants. This diagram divides the RH/T relationship into three periods of the year: spring-autumn, summer and winter.

The temperature measurement is not carried out inside the casing of the device, since the sensing is performed through a thermopile sensor, based on the reflection of the infrared spectrum of the matter facing it: air, floors, ceilings, furniture and people. This sensor is capable of making a pseudo thermal digital image of the entire room in front of it. A microprocessor internal to the sensor continuously runs an algorithm resulting in an average temperature of the volume of the room being measured. This type of sensor, called thermopiles, was technologically developed and popularised thanks to the manufacture of tympanic thermometers in order to faithfully measure body temperature. Its operation is based on the studies of Leopoldo Nobili (1784-1835) of the thermoelectric effect when measuring infrared radiation (IR) with two Bismuth and Antimony contacts, as well as on meeting the formula for the maximum wavelength emitted by the bodies, by Wilhelm Wien (1864-1928):

$$\lambda_{max} = 2898/T$$

$\lambda$: Wavelength in $\mu$m; T: Temperature in K (Kelvin)

The appearance of Planck's law (Max Planck 1858 - 1947) on the radiation of a black body, where it is stated that it absorbs all wavelengths, emitting only infrared radiation representative of its temperature, was the last necessary theoretical link so that this technology could be developed. The construction of this type of sensor consists of: a metal capsule containing a Bismuth-Antimony (Bi-Sb) substrate, a special filter for the radiation that is intended to be absorbed (IR) and a microprocessor circuit. The Bi-Sb substrate is a matrix of rows and columns, which can reach (depending on the manufacturer's model) up to 200 cells, each offering its own measurement. These measurements are managed by the microprocessor internal to the sensor to offer a final measurement at regular intervals (adjustable depending on the manufacturers). The result is a sensor that reports the temperature of the matter facing it, regardless of whether it is a solid or a gas, such as Nitrogen. In the case of gases, a correction factor is applied depending on the type of gas since, for example, Neon only emits 62% IR radiation, while Nitrogen emits 100%. It is therefore ideal for measuring the temperature of the surrounding air, as it contains 80% Nitrogen. The aperture angle of the measurement window ranges from a 10° cone (ideal for tympanic thermometers) to 90°, ideal for measuring large rooms, as is the case here.

The digital outputs intended for the actuation thereof depending on the current level: 1,2,3,4 or 5, are mainly intended to start ventilating and purifying at the moment in which the level changes from normal level to average level, although the final use of these contacts is left to the free choice of the user or final installer. The digital output 1 is actuated whenever any of the variables crosses the threshold from normal to average, in order to move the air in the room. However, the digital output 2 is not actuated if it is only the CO level$_2$ that has crossed over to an average level. This output is therefore actuated if the change from normal to average is due to VOC or PM. When digital output 2 is actuated, the symbol of the white stars lights up on the front interface to inform the user that purification is being performed. In this way, air quality is improved by renewing it by ventilation, totally or partially coming from the exterior with the digital output 1 and is purified with the digital output 2 actuating ionisers, hydrogen peroxide generators, ozonisers, active static electricity filters, etc.

The analogue output consists of a typical 0 to 10 volt output and is implemented to be able to maintain, increase or decrease the air flow to be renewed and/or purified depending on the current level. By default, in the excellent and normal levels, a constant value of 1.5V is maintained to maintain a minimum level of ventilation, required by most standards in European countries, even if there is an excellent or normal level of air quality. In the average level range, a voltage of 3.3 volts is imposed, in the poor level range a voltage of 6.6V is applied to this output and in the bad level interval 9.9 volts are applied.

The parametric values that determine the level changes (colours), the voltage values of the analogue output at each level and the RGB values of the colours of each level can be modified by accessing the corresponding Modbus registers using any of the many free programmes which are accessible on the Internet.

These parameters are preset by default, in 5 classifications, from best to worst: 1=Excellent, 2=Good, 3=Average, 4=Poor and 5=Bad. An identifying colour corresponds to these five classifications, namely: Blue, Green, Yellow, Orange and Red, respectively. The correspondence between the classifications and parametric values of the variables are the following:

Table of classifications vs parametric values.

| LEVEL | COLOUR | PM1 $\mu g/m^3$ | PM2.5 $\mu g/m^3$ | PM10 $\mu g/m^3$ | VOC ppb | $CO_2$ ppm |
|---|---|---|---|---|---|---|
| EXCELLENT | BLUE | (0.8] | (0.10] | (0.20] | (0.93] | (0.550] |
| NORMAL | GREEN | (8.16] | (10.20] | (20.40] | (93.310] | (550.800] |
| AVERAGE | YELLOW | (16.20] | (20.25] | (40.50] | (310.929] | (800.1300] |
| POOR | ORANGE | (20.40] | (25.50] | (50.100] | (929.3097] | (1300.2500] |
| BAD | RED | (40,∞] | (50,∞] | (100,∞] | (3097,∞] | (2500,∞] |

To determine the level of comfort and based on the ASHRAE 55-1992 diagram:

The space of the quadrant of the diagram is divided into 5 zones, characterising the central core of the shading of the table as the most comfortable (1= Excellent = Blue), to the least good (5 = Bad = Red). The overlapping area between winter and summer is considered spring-autumn, therefore having 3 slightly different divisions:
TEMPERATURE °C SPRING-AUTUMN

| RH % | <18 | 18.5 | 19.5 | 21.5 | 22.5 | 23 | 23.5 | 23.7 | 23.8 | 24 | 24.5 | 25 | 26 | 27 | 29 | >31 |
|---|---|---|---|---|---|---|---|---|---|---|---|---|---|---|---|---|
| >86.5 | 5 | 5 | 5 | 5 | 5 | 5 | 5 | 5 | 5 | 5 | 5 | 5 | 5 | 5 | 5 | 5 |
| 86.5 | 5 | 4 | 4 | 4 | 4 | 4 | 4 | 4 | 4 | 4 | 4 | 4 | 4 | 4 | 4 | 5 |
| 79.5 | 5 | 4 | 3 | 3 | 3 | 3 | 3 | 3 | 3 | 3 | 3 | 3 | 3 | 3 | 4 | 5 |
| 58.3 | 5 | 4 | 3 | 2 | 2 | 2 | 2 | 2 | 2 | 2 | 2 | 2 | 3 | 3 | 4 | 5 |
| 57.3 | 5 | 4 | 3 | 2 | 2 | 2 | 2 | 2 | 2 | 2 | 2 | 2 | 3 | 3 | 4 | 5 |
| 50 | 5 | 4 | 3 | 2 | 2 | 1 | 1 | 1 | 1 | 1 | 2 | 2 | 3 | 3 | 4 | 5 |
| 45 | 5 | 4 | 3 | 2 | 2 | 1 | 1 | 1 | 1 | 1 | 2 | 2 | 3 | 3 | 4 | 5 |
| 40 | 5 | 4 | 3 | 2 | 2 | 1 | 1 | 1 | 1 | 1 | 2 | 2 | 3 | 3 | 4 | 5 |
| 35 | 5 | 4 | 3 | 2 | 2 | 1 | 1 | 1 | 1 | 1 | 2 | 2 | 3 | 3 | 4 | 5 |
| 30 | 5 | 4 | 3 | 2 | 2 | 1 | 1 | 1 | 1 | 1 | 2 | 2 | 3 | 3 | 4 | 5 |
| 29.3 | 5 | 4 | 3 | 2 | 2 | 2 | 2 | 2 | 2 | 2 | 2 | 2 | 3 | 3 | 4 | 5 |
| 24.4 | 5 | 4 | 3 | 2 | 2 | 2 | 2 | 2 | 2 | 2 | 2 | 2 | 3 | 3 | 4 | 5 |
| 20 | 5 | 4 | 3 | 3 | 3 | 3 | 3 | 3 | 3 | 3 | 3 | 3 | 3 | 3 | 4 | 5 |
| 19.8 | 5 | 4 | 4 | 4 | 4 | 4 | 4 | 4 | 4 | 4 | 4 | 4 | 4 | 4 | 4 | 5 |
| <19.8 | 5 | 5 | 5 | 5 | 5 | 5 | 5 | 5 | 5 | 5 | 5 | 5 | 5 | 5 | 5 | 5 |

TEMPERATURE °C SUMMER

| RH % | <18 | 18.5 | 19.5 | 21.5 | 22.5 | 23 | 23.5 | 23.7 | 23.8 | 24 | 24.5 | 25 | 26 | 27 | 29 | >31 |
|---|---|---|---|---|---|---|---|---|---|---|---|---|---|---|---|---|
| >86.5 | 5 | 5 | 5 | 5 | 5 | 5 | 5 | 5 | 5 | 5 | 5 | 5 | 5 | 5 | 5 | 5 |
| 86.5 | 5 | 4 | 4 | 4 | 4 | 4 | 4 | 4 | 4 | 4 | 4 | 4 | 4 | 4 | 4 | 5 |
| 79.5 | 5 | 4 | 4 | 3 | 3 | 3 | 3 | 3 | 3 | 3 | 3 | 3 | 3 | 3 | 4 | 5 |
| 58.3 | 5 | 4 | 4 | 3 | 3 | 3 | 3 | 3 | 3 | 3 | 3 | 3 | 3 | 3 | 4 | 5 |
| 57.3 | 5 | 4 | 4 | 3 | 2 | 2 | 2 | 2 | 2 | 2 | 2 | 2 | 2 | 3 | 4 | 5 |
| 50 | 5 | 4 | 4 | 3 | 2 | 2 | 2 | 2 | 2 | 2 | 2 | 2 | 2 | 3 | 4 | 5 |
| 45 | 5 | 4 | 4 | 3 | 2 | 2 | 1 | 1 | 1 | 1 | 1 | 2 | 2 | 3 | 4 | 5 |
| 40 | 5 | 4 | 4 | 3 | 2 | 2 | 1 | 1 | 1 | 1 | 1 | 2 | 2 | 3 | 4 | 5 |
| 35 | 5 | 4 | 4 | 3 | 2 | 2 | 1 | 1 | 1 | 1 | 1 | 2 | 2 | 3 | 4 | 5 |
| 30 | 5 | 4 | 4 | 3 | 2 | 2 | 1 | 1 | 1 | 1 | 1 | 2 | 2 | 3 | 4 | 5 |
| 29.3 | 5 | 4 | 4 | 3 | 2 | 2 | 1 | 1 | 1 | 1 | 1 | 2 | 2 | 3 | 4 | 5 |
| 24.4 | 5 | 4 | 4 | 3 | 2 | 2 | 2 | 2 | 2 | 2 | 2 | 2 | 2 | 3 | 4 | 5 |
| 20 | 5 | 4 | 4 | 3 | 3 | 3 | 3 | 3 | 3 | 3 | 3 | 3 | 3 | 3 | 4 | 5 |
| 19.8 | 5 | 4 | 4 | 4 | 4 | 4 | 4 | 4 | 4 | 4 | 4 | 4 | 4 | 4 | 4 | 5 |
| <19.8 | 5 | 5 | 5 | 5 | 5 | 5 | 5 | 5 | 5 | 5 | 5 | 5 | 5 | 5 | 5 | 5 |

TEMPERATURE °C WINTER

| RH % | <18 | 18.5 | 19.5 | 21.5 | 22.5 | 23 | 23.5 | 23.7 | 23.8 | 24 | 24.5 | 25 | 26 | 27 | 29 | >31 |
|---|---|---|---|---|---|---|---|---|---|---|---|---|---|---|---|---|
| >86.5 | 5 | 5 | 5 | 5 | 5 | 5 | 5 | 5 | 5 | 5 | 5 | 5 | 5 | 5 | 5 | 5 |
| 86.5 | 5 | 4 | 4 | 4 | 4 | 4 | 4 | 4 | 4 | 4 | 4 | 4 | 4 | 4 | 4 | 5 |
| 79.5 | 5 | 4 | 3 | 3 | 3 | 3 | 3 | 3 | 3 | 3 | 3 | 3 | 3 | 4 | 4 | 5 |
| 58.3 | 5 | 4 | 3 | 2 | 2 | 2 | 2 | 2 | 2 | 2 | 2 | 3 | 3 | 4 | 4 | 5 |
| 57.3 | 5 | 4 | 3 | 2 | 1 | 1 | 1 | 1 | 1 | 2 | 2 | 3 | 3 | 4 | 4 | 5 |
| 50 | 5 | 4 | 3 | 2 | 1 | 1 | 1 | 1 | 1 | 2 | 2 | 3 | 3 | 4 | 4 | 5 |
| 45 | 5 | 4 | 3 | 2 | 1 | 1 | 1 | 1 | 1 | 2 | 2 | 3 | 3 | 4 | 4 | 5 |
| 40 | 5 | 4 | 3 | 2 | 1 | 1 | 1 | 1 | 1 | 2 | 2 | 3 | 3 | 4 | 4 | 5 |
| 35 | 5 | 4 | 3 | 2 | 1 | 1 | 1 | 1 | 1 | 2 | 2 | 3 | 3 | 4 | 4 | 5 |
| 30 | 5 | 4 | 3 | 2 | 2 | 2 | 2 | 2 | 2 | 2 | 2 | 3 | 3 | 4 | 4 | 5 |
| 29.3 | 5 | 4 | 3 | 2 | 2 | 2 | 2 | 2 | 2 | 2 | 2 | 3 | 3 | 4 | 4 | 5 |
| 24.4 | 5 | 4 | 3 | 3 | 3 | 3 | 3 | 3 | 3 | 3 | 3 | 3 | 3 | 4 | 4 | 5 |
| 20 | 5 | 4 | 3 | 3 | 3 | 3 | 3 | 3 | 3 | 3 | 3 | 3 | 3 | 4 | 4 | 5 |
| 19.8 | 5 | 4 | 4 | 4 | 4 | 4 | 4 | 4 | 4 | 4 | 4 | 4 | 4 | 4 | 4 | 5 |
| <19.8 | 5 | 5 | 5 | 5 | 5 | 5 | 5 | 5 | 5 | 5 | 5 | 5 | 5 | 5 | 5 | 5 |

The resulting level of comfort is not reflected visually on the device itself, but is displayed unequivocally in the Internet-accessible application associated with said device. This application connects to a cloud where the level of comfort is calculated based on relative humidity, temperature and date of the year, such that the algorithm uses one of the three tables above. This information, its calculation and classification, is shown to the user both descriptively and in the form of colours, being a function of the sensed temperature and relative humidity variables.

On the front of the device, four variables will be displayed using a circle of RGB light. The display of the one that is measured is executed by switching in turn, by pressing the lower button. The first variable represents a general assessment, referred to using the acronym AQ, and shown with a colour of the five available colours equivalent to the worst of the other three. The second variable is the particulate matter, called PM. The device has the capacity to measure 3 sizes of particulate matter, the worst of the three being the one that defines the colour to be displayed. The third variable is the parts per billion (ppb) of volatile organic compounds measured by the device, referred to using the acronym VOC. The fourth variable is $CO_2$, (Carbon dioxide), measured by the device in parts per million and referred to in the device as CO2. These five colours are the ones that will be displayed on the front of the device. The variables PM1, PM2.5, PM10, VOC, $CO_2$, Temperature and Relative Humidity are simultaneously captured in the device of the present invention.

The relationship between the measured variables and the consequent actuation of the 3 active outputs, in such a way that a degradation of the air quality is resolved in the minimum possible time due to the actuators, is the following: Any of the PM or VOC variables activate the two digital outputs simultaneously when the status changes from Normal status (green) to Average status (yellow), or worse. Changing from Average to Normal deactivates them. The firmware of the device contains a hysteresis of the values to avoid switching between regressive values, with 5 units in each band.

The $CO_2$ variable only activates the first digital output when changing from Normal (green) to Average (yellow) or worse. Changing from Average to Normal deactivates it. The analogue output provides a constant voltage of 1.5V in its Excellent and Normal levels, in order to maintain constant ventilation even when the air surrounding the device is of good quality. Any of the variables (PM, VOC or $CO_2$) that change to the Average state will impose an analogue output voltage of 3.3V. Likewise, if one of them changes to the Poor state (Orange), the analogue output voltage will rise to 6.6V. Ultimately, if any of them were in the Bad state (Red), the analogue output voltage will be 9.9V. These voltages are applied to ventilation machines or air conditioning machines or heat recovery machines that have an input of 0 to 10V to control the air flow, 0V typically being the shut-down machine and 10V at flow.

The main strategy for restoring the air quality in the event of degradation is: ventilate only, if $CO_2$ is the variable that causes this degradation (activating a single digital output). Ventilate and purify by activating the two digital outputs, one to ventilate and the other to activate a purifier, the combination of a hydrogen peroxide generator with ionizers such as flocculants being highly recommended, all with a small percentage of ozone ($O_3$) less than 0.01ppm to ensure its safety.

As a complementary strategy, controlling the flow of air forced by machines that have control from 0 to 10V is available, such that, the greater the degradation of the air, the greater the flow.

Except when indicated otherwise, all of the technical and scientific elements used in this specification have the meaning commonly understood by a person with average skill in the art to which this invention belongs. When this invention is put into practice, methods and materials may be used that are similar or equivalent to the ones described in the specification.

Throughout the description and the claims, the word "comprise" and its variants are not intended to exclude other technical features, additions, components or steps. For those skilled in the art, other objects, advantages and features of the invention will be deduced from both the description and the practical use of the invention.

## DESCRIPTION OF THE FIGURES

As a complement to the description provided herein, and for the purpose of helping to make the features of the invention more readily understandable, in accordance with a preferred practical exemplary embodiment thereof, said description is accompanied by a set of drawings which, by way of illustration and not limitation, represent the following.

Figure 1 shows the front view (1) and the rear face (1') of the device of the invention.

Figure 2 shows a general representation of a complete installation of the assembly with all its connection possibilities.

Figure 3 shows an exploded view of the device of the invention. Detail A is included to show the internal recirculation of the air in a bottom-up direction.

The numbered elements of the figures are listed below:

1- Device that is the subject matter of the invention application in its front perspective, which covers several claims.
1' - Device subject matter of the invention application in a rear perspective thereof.
2- Connection to conventional 230Vac electrical grid. Quick connection for input and output of the pressure wires, of the external power supply to the conventional 230Vac electrical grid.
3- ModBus connection. Quick connection for input and output of pressure wires, of A+Band COM terminals to communicate via ModBus protocol.
4- Connection A, B, COM. Quick connection of input and output of the pressure wires, of the A+B- and COM terminals to be used as an output and communicate via ModBus protocol with other devices via ModBus protocol in Daisy Chain topology.

5- First digital output. Quick connection for input and output of pressure wires. It consists of a simple potential-free contact the purpose of which is to act on a machine that ventilates or renews the air in the room. Its closure is actuated when the CO2 level exceeds level 3 (or higher) or changes from green to yellow in the front circle (21). It is also actuated when the VOC or PM levels go from level 3 (when changing from green to yellow in the central circle of the device of the invention).

6- Second digital output. Quick connection for input and output of pressure wires. It consists of a double contact with the indication regarding a common contact of the NO and NC positions (acronyms for Normally Open and Normally Closed). Its use is intended for devices capable of purifying and freeing the surrounding air in the room from substances harmful to human health. It is actuated when the VOC or PM levels change to level 3 or higher (when the colour changes from green to yellow in the central circle of the device of the invention).

7- Analogue output. Quick connection for input and output of pressure wires, type 0-10V. Its purpose is to regulate the flow rate of the air impeller machine, in cases where said machine has this control input. It is an output that offers a direct voltage with polarity, configured such that there is a minimum of 1.5 volts, ensuring a minimum flow rate to maintain the aeration of the room, increasing to levels 3, 4 and 5, 3.3V, 6.6V and 9.9V are imposed respectively.

8- Digital input 1. Quick connection for input and output of the pressure wires, which when joined with GND informs the APP that pressure has been lost in the air ducts.

8'- Digital input 2. Quick connection for input and output of the pressure wires, which when joined with GND deactivates the output contacts DO1 and DO2. This causes a shut-down of machines.

9- Central portion LED diffuser. RGB light transmitter to report the levels, offering the colours blue, green, yellow, orange and red. If illuminated with white light, a special status of the device of the invention is reported.

10- Star icon. They light up white when the VOC or PM values exceed levels 2, changing to level 3, or they change from green to yellow.

11- Push button. Momentarily pressing it switches in turn the variable to be displayed: AQ, PM, VOC or $CO_2$.

12- Light icons. They illuminate in white by means of white LED light diffusion of the figures AQ, PM, VOC or CO2.

13- Polycarbonate front. It performs the functions of diffuser for the LED indicators and allows the lower entry and upper exit of the air.

14- ABS plastic casing. It contains the air circulation chambers, prevents light pollution between the various LED indicators and supports the electronic unit.

15- Electronic unit that contains the HW of the device of the invention, including the sensors and quick connectors.

16- Rear housing of the casing with the necessary opening to access the connectors and capable of fitting into the wall support.

17- Wall support. Screwing to any wall using 2 rawl plugs and 2 screws allows the device to be secured due to 4 tabs that are push fitted into place and it is released by extraction pressure. It offers a central opening for the exit of the wiring used.

18- Thermopile sensor. It measures the overall temperature of the room.

19- Air impeller machine, which can be an air conditioning machine, forced ventilation, a heat recovery machine, etc.

20- Active air purification device, which can be a hydrogen peroxide emitter, electrostatic particle filters, ionisers, ozone generator, etc.

21- Pressure switch. External pressure sensor that closes a normally open contact when pressure is lost in the duct.

22- Normally open external contact that, when closed, the implication in the assembly is that a general shut down must be carried out.

23- Conventional 230Vac electrical grid, which can also be a power wire for the energy source of the external element.

24- Router with 2.4GHz Wi-Fi connectivity.

25- Global Internet, WWW.

26- Devices of the invention connected by Wi-Fi, integrated into the same application, and can be installed in the same location or in different locations and with no limit on quantity, as long as they have Wi-Fi coverage.

## PREFERRED EMBODIMENT OF THE INVENTION

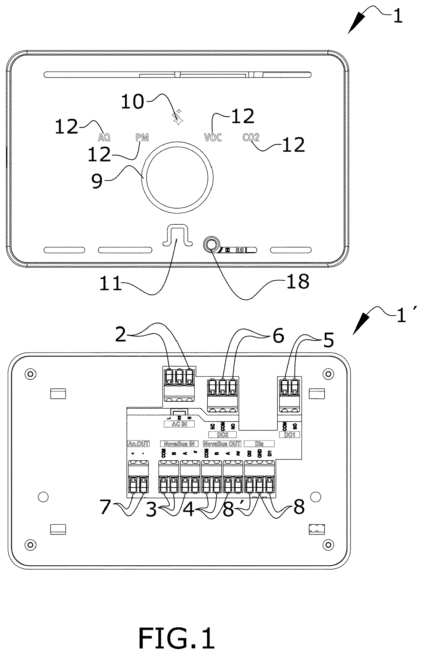

Figure 1 shows the anterior or front face (1) and the rear face (1') of the sensor-actuator device.

On the rear face (1') it can be seen that the device comprises:

- A first digital output (DO1) (5) which is a normally open contact
- A second digital output (DO2) (6) which is a contact that offers normally open and closed switching with respect to a common switching.
- An analogue output (7) that offers a direct voltage in a range of 0 to 10Vdc,

- A first digital input (8) that, when connected to ground, interacts with the device to detect if there is pressure in the ducts (through a pressure switch). If there is no pressure in the air ducts, the device receives a shut-off for DI1 with GND and will report in the APP, warning of this event.
- A second digital input (8') that when connected to ground opens the contacts (DO1) and (DO2).
- A RS485 serial port (3) with ModBus protocol.
- An output (4) for possible Daisy Chain connectivity.
- A micro USB type B female connector (2);

On the front face (1) there is a series of air quality indicator means or man-machine interface and which comprises:

- A star icon (10) is used to inform that the second digital output (DO2) has been activated since a certain level 3 or higher of VOC or PM has been reached, that is, the surrounding atmosphere of the device is being purified.
- A front portion with a series of acronyms (12), specifically AQ, PM, VOC and CO2 that are selected and highlighted in white.
- A central portion (9), which shows different colours and depending on the colour shown, has a different meaning. Specifically:

    ◦ Level 1: blue colour considered excellent.
    ◦ Level 2: green colour considered normal.
    ◦ Level 3: yellow colour considered average.
    ◦ Level 4: orange colour considered poor.
    ◦ Level 5: red colour considered bad.

- A push button (11) that allows the different values that are measured to be consecutively selected: AQ, PM, VOC and CO2.
- A thermopile sensor (18) that measures the global temperature of the room.

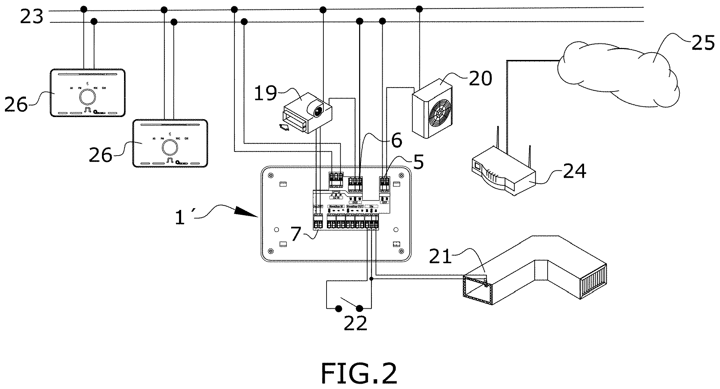

Figure 2 shows the device subject matter of the invention and its connection with different apparatus that allow global operation. On the one hand, the device is capable of being connected with:

- an air impeller machine (19) that, through the analogue output (7), which is a typical 0 to 10 volt output, allows the air flow to be renewed and/or purified depending on the current level to be maintained, increased or decreased,
- with an air purification machine (20).
- A pressure switch (21) or pressure sensor that closes a normally open contact when pressure is lost in the duct.
- A normally open external contact (22) that when closed means that a general shut down must be carried out.
- A router (25) with Wi-Fi connectivity with the global Internet network (25).

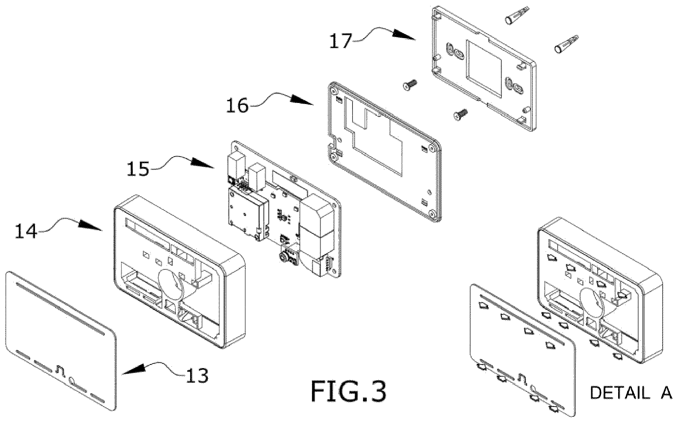

In figure 3 it can be seen that the device consists of 4 main elements: polycarbonate front (13) which, apart from being the front, acts as a light diffuser; main housing (14) that encloses the air recirculation chambers and prevents the transmission of light between the different sections of LED lighting; electronic unit (15), where the digital and analogue circuitry, connectors and sensors are integrated; rear housing (16) that protects the rear face of the electronic unit, showing access only to the external connectors. This rear housing can be inserted by means of tabs into an additional piece (17), for wall installation. The casing is designed internally in such a way that air recirculation from bottom to top by natural convection is favoured (DETAIL A, Figure 3), with the sampling constantly being renewed, for a good sensing of the variables.

The connection of the external devices must be done by closing the normally open contacts of the relays of the device corresponding to the outputs DO1 (5) and DO2 (6). The use of the outlet DO1 for ventilation and the outlet DO2 for purification is highly recommended. The connection of the digital inputs is intended to protect the installation from anomalies or cause a general outage for whatever reason. The connection of the RS485 serial port under the ModBus protocol will be implemented when multiple devices are to be connected, and can be integrated into a BMS system as a master, with the devices being slaves. The wiring of this connection must be done with standard RS485 shielded and twisted cables in pairs, where wires A+ and B- are a twisted pair and the GND wire is connected to the shield of the cable. The device provides an input connection, which is a RS485 serial port (3) and another output connection (4) to facilitate the Daisy Chain topology. During the installation and by means of the computer application, the device furthest away in distance is activated as the one that electronically activates the 120Ω ohmic terminal, to ensure the quality of the serial data. If the external element responsible for impelling the air contains a 0-10V input, which regulates from lower to higher voltage, the air flow from lower to higher, the analogue output (7) of the device can be connected to this input, modulating in this way the flow rate necessary to ventilate depending on the level of deterioration in air quality, that is, the higher the deterioration in air quality, the greater the impeller flow. If the device is used by means of an application on a smartphone, tablet or similar, the integrated Wi-Fi module must be activated by reading the QR code affixed to the back housing. The Internet connection is executed using WPS with the router to be connected or by inserting the router data (router identifier and password) in the application. Using the same method, an unlimited number of devices (26) can be registered in the same application installed on the user's smartphone, and must have coverage with a router. In this way, said user could, for example, control the air quality of their office, workshop, home, etc., in the same application.

**Claims**

1. An air quality sensor-actuator device that measures different variables concerning the air **characterised in** it comprises:

    - a series of sensor means,
    - processing means to carry out a series of calculations of a series of variables and, based on the data obtained, send command and actuation signals on external devices,
    - a man-machine interface for displaying of the quality of the different measured parameters, and
    - means for connectivity with the exterior through a wireless connection,
    wherein the sensor means that the device subject matter of the invention has are:

        - a temperature sensor,
        - a relative humidity sensor,
        - a particulate matter sensor that measures particle sizes of 1 micron, 2.5 microns and 10 microns,
        - another sensor for measuring volatile organic compounds (VOC) and
        - a fifth sensor for measuring CO2,

    and the variables calculated by means of the processing means are: absolute humidity, dew point and thermal comfort.

2. The air quality sensor-actuator device according to claim 1 **characterised in that** the device internally comprises:

    - A first digital output (DO1) (5) which is a normally open contact,
    - A second digital output (DO2) (6) which is a contact that offers normally open and closed switching with respect to a common switching,
    - An analogue output (7) that offers a direct voltage in a range of 0 to 10Vdc,
    - A first digital input (8) that, when connected to ground, interacts with the device to detect if there is pressure in the ducts (through a pressure switch). If there is no pressure in the air ducts, the device receives a shut-off for DI1 with GND and will report in the APP, warning of this event,
    - A second digital input (8') that when connected to ground opens the contacts (DO1) and (DO2),
    - A RS485 serial port (3) with ModBus protocol,
    - An output (4) for possible Daisy Chain connectivity,
    - A micro USB type B female connector (2).

3. The air quality sensor-actuator device according to claim 1 or 2 **characterised in that** the human-machine interface for displaying the quality of the different measured parameters comprises:

    - An icon (10) to inform that the second digital output (DO2) has been activated since a certain level 3 or higher of VOC or PM has been reached, that is, the surrounding atmosphere of the device is being purified,
    - A front portion with a series of acronyms (12), specifically AQ, PM, VOC and CO2 that correspond to air quality, particle size, volatile organic compounds, and $CO_2$ level respectively, which are selected and highlighted in white,
    - A central portion (9), which in the shown embodiment is circular, which shows different colours and depending on the colour shown has a different meaning,
    - A push button (11) that allows the different values that are measured to be consecutively selected: AQ, PM, VOC and CO2,
    - A thermopile sensor (18) that measures the global temperature of the room.

4. The air quality sensor-actuator device according to claim 3, **characterised in that** the different colours shown in the central portion (9) have the following colour and meaning.

  ◦ Level 1: blue colour considered excellent.
  ◦ Level 2: green colour considered normal.
  ◦ Level 3: yellow colour considered average.
  ◦ Level 4: orange colour considered poor.
  ◦ Level 5: red colour considered bad.

5. The air quality sensor-actuator device according to any of the preceding claims, **characterised in that** the device is capable of connecting with:

  - an air impeller machine (19) that, through the analogue output (7), which is a typical 0 to 10 volt output, allows the air flow to be renewed and/or purified depending on the current level to be maintained, increased or decreased,
  - with an air purification machine (20).
  - A pressure switch (21) or pressure sensor that closes a normally open contact when pressure is lost in the duct.
  - A normally open external contact (22) that when closed means that a general shut down must be carried out.

6. The air quality sensor-actuator device according to any of the preceding claims, **characterised in that** the device is capable of connecting wirelessly with:

  - A router (25) with Wi-Fi connectivity with the global Internet network (25).

7. The air quality sensor-actuator device according to any of the preceding claims, **characterised in that** the device comprises four main elements: polycarbonate front (13) which, apart from being the front, acts as a light diffuser; main housing (14) that encloses the air recirculation chambers and prevents the transmission of light between the different sections of LED lighting; electronic unit (15), where the digital and analogue circuitry, connectors and sensors are integrated; rear housing (16) that protects the rear face of the electronic unit, showing access only to the external connectors where the rear housing can be inserted by means of tabs in an additional piece (17) for wall installation.

FIG.1

FIG.2

FIG.3

DETAIL A

13

14

15

16

17

| **INTERNATIONAL SEARCH REPORT** | International application No. |
|---|---|
| | PCT/ES2022/070013 |

**A. CLASSIFICATION OF SUBJECT MATTER**

**See extra sheet**

According to International Patent Classification (IPC) or to both national classification and IPC

**B. FIELDS SEARCHED**

Minimum documentation searched (classification system followed by classification symbols)
G05B, H04L

Documentation searched other than minimum documentation to the extent that such documents are included in the fields searched

Electronic data base consulted during the international search (name of data base and, where practicable, search terms used)

EPODOC, INVENES

**C. DOCUMENTS CONSIDERED TO BE RELEVANT**

| Category* | Citation of document, with indication, where appropriate, of the relevant passages | Relevant to claim No. |
|---|---|---|
| Y | ES 2781873T T3 (DELOS LIVING LLC) 08/09/2020, Page 26, line 46 - page 27, line 49; page 12, line 10-20; page 5, line 27 and line 61. figure 7. | 1-7 |
| Y | ES 2300231 A1 (INGELABS S L) 01/06/2008, claim 10 | 1-7 |
| A | Manual de instalador. Módulo domótico de zona 100-mdz000; A3201203. 31/06/2019, 31/06/2019 [on line] [retrieved on 31/06/2019]. Retrieved of <URL: https://koolnova.com/wp-content/uploads/2019/07/A3201205-Manual-of-Instalador-M%C3%B3dulo-Dom%C3%B3tico-of-Zona-100-MDZ000-123-00165.pdf> the whole document. | 1-7 |
| A | WO 2015105763 A1 (OPENTV INC) 16/07/2015, Claims and figures. | 1-7 |

☒ Further documents are listed in the continuation of Box C.          ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 23/05/2022 | **(26/05/2022)** |
| Name and mailing address of the ISA/ | Authorized officer |
| | G. Foncillas Garrido |
| OFICINA ESPAÑOLA DE PATENTES Y MARCAS Paseo de la Castellana, 75 - 28071 Madrid (España) Facsimile No.: 91 349 53 04 | Telephone No. 91 3493282 |

Form PCT/ISA/210 (second sheet) (January 2015)

# EP 4 455 810 A1

<table>
<tr><th colspan="2">INTERNATIONAL SEARCH REPORT</th><th colspan="2">International application No.<br>PCT/ES2022/070013</th></tr>
<tr><td colspan="4">C (continuation).       DOCUMENTS CONSIDERED TO BE RELEVANT</td></tr>
<tr><td>Category *</td><td colspan="2">Citation of documents, with indication, where appropriate, of the relevant passages</td><td>Relevant to claim No.</td></tr>
<tr><td>A</td><td colspan="2">ES 2654642T T3 (DAIKIN IND LTD   ET AL.) 14/02/2018,<br>claims and figures.</td><td>1-7</td></tr>
</table>

Form PCT/ISA/210 (continuation of second sheet) (January 2015)

## INTERNATIONAL SEARCH REPORT

Information on patent family members

| International application No. |
|---|
| PCT/ES2022/070013 |

| Patent document cited in the search report | Publication date | Patent family member(s) | Publication date |
|---|---|---|---|
| ES2781873T T3 | 08.09.2020 | US2021035686 A1 | 04.02.2021 |
| | | EP3702685 A1 | 02.09.2020 |
| | | US2019310670 A1 | 10.10.2019 |
| | | US10928842 B2 | 23.02.2021 |
| | | BR112015004595 A2 | 04.07.2017 |
| | | CN106950908 A | 14.07.2017 |
| | | US2017139386 A1 | 18.05.2017 |
| | | US10845829 B2 | 24.11.2020 |
| | | US2017136206 A1 | 18.05.2017 |
| | | US10691148 B2 | 23.06.2020 |
| | | EP3297218 A1 | 21.03.2018 |
| | | EP3297218 B1 | 21.10.2020 |
| | | AU2017200995 A1 | 09.03.2017 |
| | | AU2017200995B B2 | 09.08.2018 |
| | | HK1210287 A1 | 15.04.2016 |
| | | MX2015002618 A | 22.01.2016 |
| | | MX350468 B | 07.09.2017 |
| | | JP2015534701 A | 03.12.2015 |
| | | CN104685428 A | 03.06.2015 |
| | | CN104685428B B | 01.03.2017 |
| | | AU2013308871 A1 | 19.03.2015 |
| | | AU2013308871B B2 | 13.04.2017 |
| | | CA2882537 A1 | 06.03.2014 |
| | | US2014067130 A1 | 06.03.2014 |
| | | US9715242 B2 | 25.07.2017 |
| | | EP2891019 A1 | 08.07.2015 |
| | | EP2891019 A4 | 31.08.2016 |
| | | WO2014036133 A1 | 06.03.2014 |
| ES2300231 A1 | 01.06.2008 | NONE | |
| WO 15105763 A1 | 16.07.2015 | EP3940511 A1 | 19.01.2022 |
| | | MX2016008891 A | 18.01.2017 |
| | | MX361489 B | 07.12.2018 |
| | | JP2017508195 A | 23.03.2017 |
| | | CN106104420 A | 09.11.2016 |
| | | KR20160106079 A | 09.09.2016 |
| | | SG11201605502P A | 30.08.2016 |
| | | AU2015204992 A1 | 21.07.2016 |
| | | CA2935950 A1 | 16.07.2015 |
| | | EP3092548 A1 | 16.11.2016 |
| | | EP3092548 A4 | 01.11.2017 |
| | | US2015193127 A1 | 09.07.2015 |
| ES2654642T T3 | 14.02.2018 | BR112015008407 A2 | 04.07.2017 |
| | | BR112015008407 B1 | 18.05.2021 |
| | | JPWO2014061129A1 | 28.10.2015 |
| | | JP5802339B B2 | 28.10.2015 |
| | | US2015285530 A1 | 08.10.2015 |
| | | US9410715 B2 | 09.08.2016 |

Form PCT/ISA/210 (patent family annex) (January 2015)

**INTERNATIONAL SEARCH REPORT**

Information on patent family members

| International application No. |
| --- |
| PCT/ES2022/070013 |

| Patent document cited in the search report | Publication date | Patent family member(s) | Publication date |
| --- | --- | --- | --- |
| | | CN104755849 A | 01.07.2015 |
| | | CN104755849B B | 05.04.2017 |
| | | AU2012392672 A1 | 28.05.2015 |
| | | AU2012392672B B2 | 11.06.2015 |
| | | WO2014061129 A1 | 24.04.2014 |
| | | EP2873928 A1 | 20.05.2015 |
| | | EP2873928 A4 | 22.07.2015 |

Form PCT/ISA/210 (patent family annex) (January 2015)

**INTERNATIONAL SEARCH REPORT**

International application No.

PCT/ES2022/070013

**CLASSIFICATION OF SUBJECT MATTER**

*G05B19/406* (2006.01)
*G05B19/414* (2006.01)
*G05B15/02* (2006.01)
*H04L12/28* (2006.01)

Form PCT/ISA/210 (extra sheet) (January 2015)